**Europäisches Patentamt**

**European Patent Office**

**Office européen des brevets**

(11) Publication number: **0 553 072 B1**

# EUROPEAN PATENT SPECIFICATION

(45) Date of publication of patent specification: **20.12.95**

(51) Int. Cl.⁶: **C01B 39/48**, C01B 35/12, C01G 17/00, C01G 49/00, B01J 29/04, C07C 2/66

(21) Application number: **90915896.6**

(22) Date of filing: **17.10.90**

(86) International application number: **PCT/US90/05972**

(87) International publication number: **WO 92/06921 (30.04.92 92/10)**

(54) **SYNTHETIC POROUS CRYSTALLINE MATERIAL, ITS SYNTHESIS AND USE**

(43) Date of publication of application:
**04.08.93 Bulletin 93/31**

(45) Publication of the grant of the patent:
**20.12.95 Bulletin 95/51**

(84) Designated Contracting States:
**BE DE DK FR GB IT NL SE**

(56) References cited:
**EP-A- 0 064 205**
**EP-A- 0 293 032**
**US-A- 4 925 548**

(73) Proprietor: **MOBIL OIL CORPORATION**
**3225 Gallows Road**
**Fairfax,**
**Virginia 22037-0001 (US)**

(72) Inventor: **RUBIN, Mae, Koenig**
**50 Belmont Avenue**
**Bala Cynwyd, PA 19004 (US)**

(74) Representative: **Colmer, Stephen Gary et al**
**European Office of Patent Counsel**
**Mobil House**
**500-600 Witan Gate**
**Central Milton Keynes**
**Buckinghamshire MK9 1ES (GB)**

Rank Xerox (UK) Business Services
(3.10/3.09/3.3.3)

**Description**

This invention relates to a synthetic porous crystalline material, designated MCM-35, to a method for its synthesis and to its use in catalytic conversion of organic compounds.

Zeolitic materials, both natural and synthetic, have been demonstrated in the past to have catalytic properties for various types of hydrocarbon conversion. Certain zeolitic materials are ordered, porous crystalline aluminosilicates having a definite crystalline structure as determined by X-ray diffraction, within which there are a large number of cavities which may be interconnected by channels or pores. These cavities and pores are uniform in size within a specific zeolitic material. Since the dimensions of these pores are such as to accept for adsorption molecules of certain dimensions while rejecting those of larger dimensions, these materials have come to be known as "molecular sieves" and are utilized in a variety of ways to take advantage of these properties.

Such molecular sieves, both natural and synthetic, include a wide variety of positive ion-containing crystalline aluminosilicates. These aluminosilicates can be described as a rigid three-dimensional framework of $SiO_4$ and $AlO_4$ in which the tetrahedra are cross-linked by the sharing of oxygen atoms whereby the ratio of the total aluminum and silicon atoms to oxygen atoms is 1:2. The electrovalence of the tetrahedra containing aluminum is balanced by the inclusion in the crystal of a cation, for example an alkali metal or an alkaline earth metal cation. This can be expressed wherein the ratio of aluminum to the number of various cations, such as Ca/2, Sr/2, Na, K or Li, is equal to unity. One type of cation may be exchanged either entirely or partially with another type of cation utilizing ion exchange techniques in a conventional manner. By means of such cation exchange, it has been possible to vary the properties of a given aluminosilicate by suitable selection of the cation. The spaces between the tetrahedra are occupied by molecules of water prior to dehydration.

Prior art techniques have resulted in the formation of a great variety of synthetic zeolites. The zeolites have come to be designated by letter or other convenient symbols, as illustrated by zeolite A (U.S. Patent 2,882,243), zeolite X (U.S. Patent 2,882,244), zeolite Y (U.S. Patent 3,130,007), zeolite ZK-5 (U.S. Patent 3,247,195), zeolite ZK-4 (U.S. Patent 3,314,752), zeolite ZSM-5 (U.S. Patent 3,702,886), zeolite ZSM-11 (U.S. Patent 3,709,979), zeolite ZSM-12 (U.S. Patent 3,832,449), zeolite ZSM-20 (U.S. Patent 3,972,983), ZSM-35 (U.S. Patent 4,016,245), and zeolite ZSM-23 (U.S. Patent 4,076,842).

The $SiO_2/Al_2O_3$ ratio of a given zeolite is often variable. For example, zeolite X can be synthesized with $SiO_2/Al_2O_3$ ratios of from 2 to 3; zeolite Y, from 3 to 6. In some zeolites, the upper limit of the $SiO_2/Al_2O_3$ ratio is unbounded. ZSM-5 is one such example wherein the $SiO_2/Al_2O_3$ ratio is greater than 5 and up to infinity. U.S. Patent 3,941,871 (Re. 29,948) discloses a porous crystalline silicate made from a reaction mixture containing no deliberately added alumina in the starting mixture and exhibiting the X-ray diffraction pattern characteristic of ZSM-5. U.S. Patents 4,061,724; 4,073,865 and 4,104,294 describe crystalline silicates or organosilicates of varying alumina and metal content.

In one aspect the present invention resides in a synthetic porous crystalline material designated "MCM-35", having an X-ray diffraction pattern including values set forth in Table 1 of the specification.

In a further aspect, the invention resides in a method for synthesizing the material of said one aspect comprising preparing a reaction mixture containing sources of alkali metal cations (M), a trivalent element (X) oxide, a tetravalent element (Y) oxide, hexamethyleneimine (R) and water, said trivalent element being selected from aluminum, boron, iron and gallium, said tetravalent element being selected from silicon and germanium, and said reaction mixture having a composition in terms of mole ratios within the following ranges:

| | |
|---|---|
| $YO_2/X_2O_3$ | = 50 to 200 |
| $H_2O/YO_2$ | = 10 to 100 |
| $OH^-/YO_2$ | = 0.01 to 0.2 |
| $M/YO_2$ | = 0.01 to 1.0 |
| $R/YO_2$ | = 0.1 to 1.0 |

and maintaining said reaction mixture under sufficient crystallization conditions until crystals of said material are formed.

The synthetic crystalline material MCM-35 has an X-ray diffraction pattern which is distinguished from that of other crystalline materials by the presence of the following lines:

TABLE 1

| Interplanar d-Spacing (Å) | Relative Intensity, I/Io |
|---|---|
| 15.4 ± 0.23 | mw-m |
| 9.03 ± 0.14 | mw-ms |
| 6.62 ± 0.10 | w-mw |
| 4.99 ± 0.07 | mw-m |
| 4.04 ± 0.05 | m-ms |
| 3.31 ± 0.04 | mw-m |
| 2.00 ± 0.03 | mw-m |

These X-ray diffraction data and those of the following specific examples were collected with a Philips diffraction system, equipped with a graphite diffracted beam monochromator and scintillation counter, using copper K-alpha radiation. The diffraction data were recorded by step-scanning at 0.04 degrees of two-theta, where theta is the Bragg angle, and a counting time of 4 seconds for each step. The interplanar spacings, d's, were calculated in Angstrom units (A), and the relative intensities of the lines, $I/I_o$, where $I_o$ is one-hundredth of the intensity of the strongest line, above backgound, were derived with the use of a profile fitting routine (or second derivative algorithm). The intensities are uncorrected for Lorentz and polarization effects. The relative intensities are given in terms of the symbols vw = very weak (0-5), w = weak (5-10), mw = medium weak (10-20), m = medium (20-40), ms = medium strong (40-60), s = strong (60-80) and vs = very strong (80-100). It should be understood that diffraction data listed as single lines may consist of multiple overlapping lines which under certain conditions, such as differences in crystallite size or very high experimental resolution or crystallographic changes, may appear as resolved or partially resolved multiplets. Typically, crystallographic changes can include minor changes in unit cell parameters and/or a change in crystal symmetry, without a change in topology of the structure. These minor effects, including changes in relative intensities, can also occur as a result of differences in cation content, framework composition, nature and degree of pore filling, and thermal and/or hydrothermal history.

Since MCM-35 may have a plate-like morphology, experimental or measured intensity distortions due to preferred orientation are possible. This was taken into account in the above analysis and is reflected in Table 1.

The crystalline material of this invention has a composition involving the molar relationship:

$$X_2O_3:(n)YO_2$$

wherein X is a trivalent element, such as aluminum, boron, iron and/or gallium, preferably aluminum, Y is a tetravalent element such as silicon and/or germanium, preferably silicon, and n is at least 30, usually from 40 to 200, more usually from 60 to 150. In the as-synthesized form, the material has a formula, on an anhydrous basis and in terms of moles of oxides per n moles of $YO_2$, as follows:

$$(0.1 - 0.8)M_2O(0.5 - 4)R_2O:X_2O_3:nYO_2$$

wherein R is an organic and M is alkali metal.

The M and R components are associated with the material as a result of their presence during crystallization, and are easily removed by conventional post-crystallization methods. Thus, the organic component can be removed by calcination, whereas the alkali metal cations M can be replaced, at least in part, by ion exchange with other cations. Preferred replacing cations include metal ions, hydrogen ions, hydrogen precursor, e.g., ammonium, ions and mixtures thereof. Particularly preferred cations are those which render the material catalytically active, especially for certain hydrocarbon conversion reactions. These include hydrogen, rare earth metals and metals of Groups IIA, IIIA, IVA, IB, IIB, IIIB, IVB and VIII of the Periodic Table of the Elements. A typical ion exchange technique would be to contact the synthetic material with a salt of the desired replacing cation or cations. Examples of such salts include the halides, e.g., chlorides, nitrates and sulfates.

MCM-35, when employed either as an adsorbent or as a catalyst in an organic compound conversion process should be dehydrated, at least partially. This can be done by heating to a temperature in the range of 200° to 595°C in an inert atmosphere, such as air or nitrogen and at atmospheric, subatmospheric or superatmospheric pressures for between 30 minutes and 48 hours. Dehydration can also be performed at room temperature merely by placing the material in a vacuum, but a longer time is required to obtain a sufficient amount of dehydration.

The crystalline material of the invention can be prepared from a reaction mixture containing sources of alkali metal cation, an oxide of the trivalent element X, e.g., aluminum, an oxide of the tetravalent element Y, e.g. silicon, an organic (R) directing agent in the form of hexamethyleneimine, and water, said reaction mixture having a composition, in terms of mole ratios of oxides, within the following ranges:

| Reactants | Useful | Preferred |
|---|---|---|
| $YO_2/X_2O_3$ | 50 to 200 | 50 to 150 |
| $H_2O/YO_2$ | 10 to 100 | 15 to 40 |
| $OH^-/YO_2$ | 0.01 to 0.20 | 0.02 to less than 0.10 |
| $M/YO_2$ | 0.01 to 1.0 | 0.05 to 0.3 |
| $R/YO_2$ | 0.1 to 1.0 | 0.1 to 0.5 |

In the above mixture, it is important to operate at low $OH^-/YO_2$ values when the $YO_2/X_2O_3$ is low since otherwise significant quantities of crystalline phases other than MCM-35 are produced.

Crystallization of MCM-35 can be carried out at either static or stirred conditions in a suitable reactor vessel, such as for example, polypropylene jars or teflon lined or stainless steel autoclaves. Crystallization is generally conducted at 80°C to 250°C for 24 hours to 20 days. Thereafter, the crystals are separated from the liquid and recovered.

Where $YO_2$ is silica, the silica source preferably contains at least 30 wt.% solid silica, e.g. Ultrasil (a precipitated, spray dried silica containing 90 wt.% silica) or HiSil (a precipitated hydrated $SiO_2$ containing 87 wt.% silica, 6 wt.% free $H_2O$ and 4.5 wt.% bound $H_2O$ of hydration and having a particle size of 0.02 micron). Preferably the silica source contains at least 80 wt.% solid silica, and more preferably at least 85 wt.% solid silica.

Synthesis of the MCM-35 is facilitated by the presence of at least 0.01 percent, preferably 0.10 percent and still more preferably 1 percent, seed crystals (based on total weight) of crystalline product.

MCM-35 can be used as a catalyst in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be exchanged into the composition, e.g. to the extent aluminum is in the structure, impregnated therein or intimately physically admixed therewith. Such component can be impregnated in or on to it such as, for example in the case of platinum, by treating the crystalline material with a solution containing a platinum metal containing ion. Thus, suitable platinum compounds include chloroplatinic acid, platinous chloride and various compounds containing the platinum amine complex.

In general, organic compounds such as, for example, hydrocarbons can be converted to conversion products such as, for example, lower molecular weight hydrocarbons, over a catalyst comprising the crystalline material of the invention by contact under organic compound conversion conditions including a temperature of from 100°C to 800°C, a pressure of 10 to 10000 kPa (0.1 to 100 atmospheres), a weight hourly space velocity of 0.08 to 20 $hr^{-1}$ and a hydrogen/feedstock organic, e.g. hydrocarbon, compound mole ratio of 0 (no added hydrogen) to 100.

Such conversion processes include, as non-limiting examples, cracking hydrocarbons to lower molecular weight hydrocarbons with reaction conditions including a temperature of 300°C to 800°C, a pressure of 10 to 3500 kPa (0.1 to 35 atmospheres) and a weight hourly space velocity of 0.1 to 20; and dehydrogenating hydrocarbon compounds with reaction conditions including a temperature of 300°C to 700°C, a pressure of 10 to 1000 kPa (0.1 to 10 atmospheres) and a weight hourly space velocity of 0.1 to 20.

More preferably, the conversion process is alkyation of one or more aromatic compounds, such as benzene, toluene, xylene and/or naphthalene, with an alkylating agent, such as an olefin. Preferably, the alkylating agent has 1 to 5 carbon atoms and most preferably is ethylene. Suitable conditions for the alkylation process include a temperature of 0 to 500°C, preferably 50 to 250°C, a pressure of 20 to 25000 kPa, preferably 100 to 2500 kPa, a WHSV of 0.1 to 500, preferably 0.5 to 100 and an aromatic/alkylating agent ratio of 0.1 to 50, preferably 0.5 t0 15.

As in the case of many catalysts, it may be desirable to incorporate MCM-35 with another material resistant to the temperatures and other conditions employed in organic conversion processes. Such materials include active and inactive materials and synthetic or naturally occurring zeolites as well as inorganic materials such as clays, silica and/or metal oxides such as alumina. The latter may be either naturally occurring or in the form of gelatinous precipitates or gels including mixtures of silica and metal oxides. Use of a material in conjunction with MCM-35, i.e. combined therewith, which is active, tends to change the conversion and/or selectivity of the catalyst in certain organic conversion processes. Inactive materials suitably serve as diluents to control the amount of conversion in a given process so that products can be obtained economically without employing other means for controlling the rate of reaction. These materials may be incorporated into naturally occurring clays, e.g. bentonite and kaolin, to improve the crush strength of the catalyst under commercial operating conditions. Said materials, i.e. clays, oxides, etc., function as binders for the catalyst. It is desirable to provide a catalyst having good crush strength because in commercial use it is desirable to prevent the catalyst from breaking down into powder-like materials. These clay binders have been employed normally only for the purpose of improving the crush strength of the catalyst.

Naturally occurring clays which can be composited with the new crystal include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with the present crystal also include inorganic oxides, notably alumina.

In addition to the foregoing materials, the new crystal can be composited with a porous matrix material such as silica-alumina, silica-magnesia, silica-zirconia, silica-thoria, silica-beryllia, silica-titania as well as ternary compositions such as silica-alumina-thoria, silica-alumina-zirconia silica-alumina-magnesia and silica-magnesia-zirconia.

The relative proportions of MCM-35 and inorganic oxide matrix vary widely, with the MCM-35 ranging from 1 to 90 percent by weight and more usually, particularly when the composite is prepared in the form of beads, in the range of 2 to 80 weight percent of the composite.

In order to more fully illustrate the nature of the invention and the manner of practicing same, the following examples are presented. In the examples all percentages are by weight, and whenever sorption data are set forth for comparison of sorptive capacities for cyclohexane, water and/or hexane, they were determined as follows:

A weighed sample of the calcined adsorbant was contacted with the desired pure adsorbate vapor in an adsorption chamber, evacuated to less than 1 mm and contacted with 40 mm Hg of of n-hexane or cyclohexane vapor, pressures less than the vapor-liquid equilibrium pressure of the respective adsorbate at 90°C. The pressure was kept constant (within ± 0.5 mm) by addition of adsorbate vapor controlled by a manostat during the adsorption period, which did not exceed 8 hours. As adsorbate was adsorbed by the absorbant, the decrease in pressure caused the manostat to open a valve which admitted more adsorbate vapor to the chamber to restore the above control pressures. Sorption was complete when the pressure change was not sufficient to activate the monastat. The increase in weight was calculated as the adsorption capacity of the sample in g/100 g of calcined adsorbant.

When Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of the highly active silica-alumina cracking catalyst taken as an Alpha of 1 (Rate Constant = 0.016 $sec^{-1}$). The Alpha Test is described in U.S. Patent 3,354,078 and in The Journal of Catalysis, Vol. IV, pp. 522-529 (August 1965).

Example 1

A 30g quantity of $Al_2(SO_4)_3.xH_2O$ was dissolved in a solution containing 56.8 g of 45% KOH and 1235 g of water. A 236 g quantity of a precipitated, spray dried silica containing 90% silica (i.e. Ultrasil) was added to the solution and the total was mixed thoroughly. Finally, 105g of hexamethyleneimine was added to the mixture with thorough agitation. The resulting reaction mixture had the following composition, in mole ratios:

| | |
|---|---|
| $Si_2/Al_2O_3$ | = 70.0 |
| $H_2O/SiO_2$ | = 19.9 |
| $OH^-/SiO_2$ | = 0.043 |
| $K/SiO_2$ | = 0.13 |
| $R/SiO_2$ | = 0.30 |

wherein R is hexamethyleneimine.

This mixture was then crystallized in a stirred reactor at 175°C for 3 days. The product crystals were recovered by filtration, washed with water and then dried at 120°C. A portion of the dried product was submitted for X-ray and chemical analysis. X-ray analysis identified the product crystals as the new crystalline material of this invention, with the X-ray powder diffraction pattern including the lines presented in Table 2.

## TABLE 2

### X-RAY DIFFRACTION PATTERN OF THE DRIED PRODUCT OF EXAMPLE 1

| Observed 2 Theta | d (Å) | Relative Intensity |
|---|---|---|
| 5.78 | 15.29 | 11 |
| 9.74 | 9.08 | 17 |
| 11.53 | 7.68 | 1 |
| 13.39 | 6.61 | 6 |
| 15.38 | 5.76 | 9 |
| 16.72 | 5.30 | 1 |
| 17.32 | 5.12 | 6 |
| 17.81 | 4.98 | 19 |
| 18.73 | 4.74 | 1 |
| 19.58 | 4.53 | 2 |
| 21.25 | 4.18 | 53 |
| 22.01 | 4.04 | 39* |
| 22.31 | 3.98 | 40 |
| 22.74 | 3.91 | 76 |
| 24.31 | 3.66 | 4 |
| 25.04 | 3.56 | 13 |
| 25.59 | 3.48 | 100 |
| 25.99 | 3.43 | 30 |
| 26.54 | 3.36 | 13** |
| 26.97 | 3.31 | 25 |
| 27.86 | 3.20 | 6 |
| 28.13 | 3.17 | 15 |
| 29.02 | 3.08 | 3 |
| 29.57 | 3.03 | 13 |
| 30.21 | 2.958 | 11 |
| 31.31 | 2.856 | 6 |
| 31.68 | 2.824 | 11 |
| 32.48 | 2.756 | 3 |

TABLE 2 (cont'd)

| Observed 2 Theta | d (Å) | Relative Intensity |
|---|---|---|
| 33.00 | 2.714 | 4 |
| 33.40 | 2.682 | 5 |
| 34.45 | 2.603 | 3 |
| 34.93 | 2.568 | 2 |
| 35.71 | 2.514 | 2 |
| 36.80 | 2.442 | 4 |
| 37.07 | 2.425 | 6 |
| 37.34 | 2.408 | 8 |
| 37.87 | 2.376 | 5 |
| 38.30 | 2.250 | 5 |
| 39.26 | 2.294 | 3 |
| 39.89 | 2.260 | 3 |
| 40.49 | 2.228 | 3 |
| 41.14 | 2.194 | 3 |
| 41.95 | 2.154 | 4 |
| 42.60 | 2.122 | 2 |
| 43.46 | 2.082 | 5 |
| 43.94 | 2.061 | 3 |
| 45.23 | 2.005 | 21* |
| 46.30 | 1.961 | 5 |
| 46.95 | 1.935 | 11 |
| 48.60 | 1.873 | 6 |
| 50.10 | 1.821 | 5 |
| 51.02 | 1.790 | 5 |
| 51.72 | 1.768 | 5 |
| 52.46 | 1.744 | 4 |
| 53.00 | 1.728 | 7 |
| 53.87 | 1.702 | 5 |
| 55.43 | 1.658 | 4 |
| 57.24 | 1.609 | 4 |
| 58.16 | 1.586 | 5 |
| 59.22 | 1.560 | 4 |

In Table 2, * indicates that a shoulder appears on the low 2 Theta side of the peak, and ** indicates that a shoulder appears on the high 2 Theta side of the peak. There was an indication of a very small amount of impurity in this sample.

The chemical composition of the product of Example 1 was as follows, in weight percent:

| | |
|---|---|
| N | 0.93 |
| K | 0.49 |
| $Al_2O_3$ | 2.4 |
| $SiO_2$ | 86.0 |
| Ash | 94.9 |
| $SiO_2/Al_2O_3$, molar | 60.9 |

A portion of the crystalline product of this example was calcined at 538°C for 6 hours in air and submitted for sorption and X-ray analysis. The X-ray powder diffraction pattern of the calcined material included the lines presented in Table 3.

## TABLE 3

## X-RAY DIFFRACTION PATTERN OF THE DRIED
## PRODUCT OF EXAMPLE 1

| Observed 2 Theta | d (Å) | Relative Intensity |
|---|---|---|
| 5.75 | 15.36 | 22 |
| 9.79 | 9.03 | 31 |
| 11.48 | 7.71 | 3 |
| 12.09 | 7.32 | 3 |
| 12.75 | 6.94 | 2 |
| 13.37 | 6.62 | 12* |
| 15.40 | 5.75 | 2 |
| 17.37 | 5.11 | 3 |
| 17.79 | 4.99 | 16 |
| 18.73 | 4.74 | 2 |
| 19.56 | 4.54 | 1 |
| 21.26 | 4.18 | 52 |
| 22.02 | 4.04 | 41* |
| 22.32 | 3.98 | 32 |
| 22.79 | 3.90 | 66 |
| 24.35 | 3.66 | 4 |
| 25.04 | 3.56 | 47 |
| 25.54 | 3.49 | 100 |
| 26.01 | 3.43 | 30 |
| 26.54 | 3.35 | 12 |
| 26.95 | 3.31 | 23** |
| 27.84 | 3.20 | 6 |
| 28.18 | 3.17 | 16 |
| 29.53 | 3.03 | 11 |
| 30.13 | 2.966 | 11 |
| 31.36 | 2.852 | 8 |
| 31.73 | 2.820 | 12 |
| 32.50 | 2.755 | 3 |
| 32.98 | 2.716 | 4 |
| 33.45 | 2.679 | 5 |
| 33.84 | 2.649 | 2 |
| 34.49 | 2.600 | 2 |
| 34.96 | 2.568 | 2 |
| 35.71 | 2.514 | 1 |
| 36.80 | 2.442 | 4 |

## TABLE 3 (cont'd)

### X-RAY DIFFRACTION PATTERN OF THE DRIED
### PRODUCT OF EXAMPLE 1

| Observed 2 Theta | d (Å) | Relative Intensity |
|---|---|---|
| 37.29 | 2.411 | 8 |
| 37.94 | 2.372 | 4 |
| 38.36 | 2.346 | 3 |
| 39.27 | 2.294 | 2 |
| 39.93 | 2.258 | 2 |
| 40.53 | 2.226 | 3 |
| 41.18 | 2.192 | 3 |
| 42.01 | 2.151 | 3 |
| 42.74 | 2.116 | 1 |
| 43.53 | 2.079 | 4 |
| 43.83 | 2.066 | 3 |
| 45.24 | 2.004 | 19* |
| 46.28 | 1.962 | 4 |
| 46.95 | 1.935 | 10 |
| 48.65 | 1.872 | 4 |
| 50.19 | 1.818 | 5 |
| 51.06 | 1.786 | 4 |
| 51.65 | 1.770 | 6 |
| 52.48 | 1.744 | 4 |
| 53.03 | 1.727 | 6 |
| 53.91 | 1.701 | 6 |
| 55.52 | 1.655 | 4 |
| 57.36 | 1.606 | 5 |
| 58.29 | 1.583 | 5 |
| 59.33 | 1.558 | 4 |

In Table 3, * indicates that a shoulder appears on the low 2 Theta side of the peak and ** indicates that a shoulder appears on the high 2 Theta side of the peak. There was one unresolved peak between d-spacings of 2.442 and 2.411 Angstroms, and the minor amount of impurity remained.

The sorption data obtained on the calcined product of this example as follows, in weight percent:

| | |
|---|---|
| Cyclohexane | 2.0 |
| n-Hexane | 2.2 |
| Water | 4.8 |

A portion of the calcined product was ammonium exchanged and calcined at 538°C for 3 hours to prepare the hydrogen form. It was then tested for catalytic activity by the Alpha test and found to have an Alpha Value of 25.

Example 2-6

Table 4 presents details of additional examples, including reaction mixture compositions, crystallization conditions, product compositions and sorption test results of calcined (6 hours at 538°C) products. Alumina was provided by $Al_2(SO_4)_3$ $xH_2O$ and silica was provided by Ultrasil in each example. Alkali metal was potassium in Examples 2, 4 and 6 and sodium in Examples 3 and 5. In Examples 3, 4 and 5 the product was substantially 100% the crystalline silicate of the present invention by X-ray analysis. The Example 2 product was found to contain trace amounts of cristobalite in addition to the material of the present

9

invention whereas the Example 6 product contained trace amounts of ZSM-5.

The X-ray diffraction patterns for the as-synthesized and calcined products of Example 2 included the values shown in Tables 5 and 6, respectively.

TABLE 4

| Example No. | 2 | 3 | 4 | 5 | 6 |
|---|---|---|---|---|---|
| Synthesis Mixture, mole ratios | | | | | |
| $SiO_2/Al_2O_3$ | 140 | 120 | 100 | 70.1 | 50.1 |
| $H_2O/SiO_2$ | 20.0 | 17.9 | 20.0 | 19.7 | 20.0 |
| $OH^-/SiO_2$ | 0.03 | 0.03 | 0.04 | 0.04 | 0.05 |
| $M/SiO_2$ | 0.05 | 0.08 | 0.10 | 0.13 | 0.17 |
| $R/SiO_2$ | 0.30 | 0.20 | 0.30 | 0.30 | 0.30 |
| Crystallization, Stirred | | | | | |
| Temp, °C/Days | 175/3 | 160/19 | 175/3 | 175/3 | 175/3 |
| Product Composition, Wt.% | | | | | |
| $SiO_2$ | 86.7 | 88.7 | 89.5 | 83.8 | 85.5 |
| $Al_2O_3$ | 1.2 | 1.4 | 1.8 | 2.3 | 3.5 |
| K | 0.37 | -- | 0.30 | -- | 0.76 |
| Na | -- | 0.16 | -- | 0.18 | -- |
| N | 0.88 | 0.96 | 0.92 | 1.21 | 0.95 |
| Ash | 88.7 | 91.0 | 91.6 | 86.8 | 93.9 |
| $SiO_2/Al_2O_3$, molar | 123 | 108 | 84.5 | 62.0 | 41.5 |
| Adsorption, Wt.% | | | | | |
| Cyclohexane | 1.7 | 3.4 | 2.2 | 2.1 | 3.4 |
| n-Hexane | 4.6 | 4.7 | 3.0 | 3.2 | 3.8 |
| Water | 3.5 | 3.2 | 3.2 | 4.9 | 6.1 |

## TABLE 5

### X-RAY DIFFRACTION PATTERN OF AS-SYNTHESIZED EXAMPLE 2 PRODUCT

| Observed 2 Theta | d (Å) | Relative Intensity |
|---|---|---|
| 5.70 | 15.50 | 23 |
| 9.73 | 9.09 | 23 |
| 11.47 | 7.71 | 1 |
| 13.38 | 6.62 | 7 |
| 15.30 | 5.79 | 10 |
| 16.65 | 5.33 | 1 |
| 17.32 | 5.12 | 7 |
| 17.76 | 4.99 | 21 |
| 18.65 | 4.76 | 1 |
| 19.49 | 4.55 | 2 |
| 21.17 | 4.20 | 60 |
| 21.94 | 4.05 | 45* |
| 22.24 | 4.00 | 32 |
| 22.74 | 3.91 | 71 |
| 24.26 | 3.67 | 2 |
| 24.95 | 3.57 | 44 |
| 25.52 | 3.49 | 100 |
| 25.95 | 3.43 | 25 |
| 26.48 | 3.37 | 12 |
| 26.90 | 3.32 | 20 |
| 27.69 | 3.22 | 6 |
| 28.06 | 3.18 | 14 |
| 28.94 | 3.08 | 3 |
| 29.48 | 3.031 | 11 |
| 30.15 | 2.964 | 9 |
| 32.95 | 2.718 | 3 |
| 31.24 | 2.863 | 5 |
| 31.61 | 2.831 | 10 |
| 32.28 | 2.765 | 2 |
| 33.38 | 2.684 | 5 |
| 34.46 | 2.610 | 2 |
| 34.89 | 2.572 | 1 |
| 35.93 | 2.500 | 3 |
| 36.70 | 2.449 | 4 |
| 37.27 | 2.413 | 7 |
| 37.86 | 2.376 | 4 |
| 38.21 | 1.356 | 2 |
| 39.82 | 2.264 | 2 |
| 40.43 | 2.231 | 2 |
| 41.12 | 2.195 | 2 |
| 41.89 | 2.157 | 4 |
| 43.37 | 2.086 | 4 |
| 45.15 | 2.000 | 20 |
| 46.24 | 1.963 | 4 |
| 46.91 | 1.937 | 10 |
| 48.53 | 1.876 | 4 |
| 50.08 | 1.821 | 4 |
| 50.94 | 1.792 | 3 |

## TABLE 5 (cont'd)

| Observed 2 Theta | d (Å) | Relative Intensity |
|---|---|---|
| 51.78 | 1.765 | 5 |
| 52.41 | 1.746 | 4 |
| 52.96 | 1.729 | 6 |
| 53.85 | 1.703 | 5 |
| 55.39 | 1.659 | 4 |
| 57.28 | 1.608 | 5 |
| 58.13 | 1.587 | 5 |
| 59.22 | 1.560 | 4 |

## TABLE 6

## X-RAY DIFFRACTION PATTERN OF CALCINED EXAMPLE 2 PRODUCT

| Observed 2 Theta | d (Å) | Relative Intensity |
|---|---|---|
| 5.73 | 15.43 | 36 |
| 9.77 | 9.05 | 40 |
| 11.44 | 7.73 | 4 |
| 12.08 | 7.33 | 4 |
| 12.69 | 6.98 | 4 |
| 13.40 | 6.61 | 17 |
| 15.33 | 5.78 | 25 |
| 17.25 | 5.14 | 3 |
| 17.78 | 4.99 | 16 |
| 18.70 | 4.75 | 2 |
| 19.54 | 4.10 | 1 |
| 21.23 | 4.18 | 60 |
| 21.75 | 4.09 | 21 |
| 21.99 | 4.04 | 43 |
| 22.32 | 3.98 | 32 |
| 22.77 | 3.91 | 61 |
| 24.31 | 3.66 | 2 |
| 24.99 | 3.56 | 45 |
| 25.58 | 3.48 | 100 |
| 25.96 | 3.43 | 24 |
| 26.51 | 3.36 | 12 |
| 26.98 | 3.31 | 25 |
| 27.73 | 3.22 | 7 |
| 28.11 | 3.17 | 16 |
| 28.99 | 3.08 | 2 |
| 29.55 | 3.02 | 13 |
| 30.09 | 2.969 | 9 |
| 31.26 | 2.861 | 7 |
| 31.66 | 2.826 | 13 |
| 32.51 | 2.754 | 4 |
| 32.99 | 2.715 | 4 |

## TABLE 6 (cont'd)

| Observed 2 Theta | d (Å) | Relative Intensity |
|---|---|---|
| 33.44 | 2.680 | 4 |
| 33.81 | 2.651 | 2 |
| 34.46 | 2.603 | 2 |
| 34.97 | 2.566 | 2 |
| 35.99 | 2.496 | 4 |
| 36.80 | 2.442 | 4 |
| 37.12 | 2.422 | 6 |
| 37.35 | 2.407 | 8 |
| 37.86 | 2.376 | 4 |
| 38.34 | 2.348 | 3 |
| 39.26 | 2.294 | 2 |
| 39.87 | 2.261 | 2 |
| 40.50 | 2.227 | 3 |
| 41.03 | 2.200 | 2 |
| 42.01 | 2.150 | 3 |
| 43.66 | 2.073 | 4 |
| 45.25 | 2.004 | 19 |
| 46.33 | 1.960 | 4 |
| 46.98 | 1.934 | 9 |
| 48.63 | 1.872 | 5 |
| 50.13 | 1.820 | 4 |
| 51.10 | 1.787 | 4 |
| 51.75 | 1.767 | 5 |
| 52.05 | 1.726 | 6 |
| 53.92 | 1.700 | 5 |
| 55.48 | 1.656 | 4 |
| 57.37 | 1.606 | 5 |
| 58.21 | 1.585 | 6 |
| 59.33 | 1.558 | 4 |

In Table 5, the * indicates that a shoulder appears on the low Theta side of the peak. In the X-ray pattern of the as-synthesized Example 2 product, there were unresolved peaks between d-spacings of 2.572 and 2.500 Angstroms and between 2.449 and 2.413 Angstroms. In the X-ray patterns of the calcined Example 2 product, there was an unresolved peak between d-spacings of 2.073 and 2.004 Angstroms.

Portions of the calcined products of Examples 2, 5 and 6 were ammonium exchanged and then calcined at 538°C for three hours to generate the hydrogen form. The resultant products gave alpha values of 2 (Example 2), 38 (Example 5) and 67 (Example 6).

Example 7

Comparative ethylbenzene synthesis reactions were run in an upflow reactor at 427°C (800°F), atmospheric pressure and a WHSV of 11 hr$^{-1}$. The molar ratio of benzene/ethylene was maintained at 10. The catalysts comprised zeolite Beta, ZSM-5 and two separate MCM-35 materials. Each catalyst was composed of 35% alumina binder and 65% zeolite. The MCM-35 materials were made in Examples 5 and 6. Each catalyst was activated by calcination in nitrogen at 540°C, followed by aqueous ammonium nitrate exchange and calcination in air at 540°C. Products of the ethylbenzene synthesis reactions included the components listed below in weight percent:

CATALYST

| Component | Beta | ZSM-5 | Example 5 MCM-35 | Example 6 MCM-35 |
|---|---|---|---|---|
| $C_2H_4$ | 0.09 | 0.19 | 0.59 | 0.25 |
| $C_3$ | 0.32 | - | - | - |
| $C_4$ | 0.40 | - | - | - |
| Benzene | 88.51 | 89.24 | 88.15 | 87.07 |
| Toluene | 4.92 | 0.39 | 0.12 | 0.18 |
| Ethylbenzene | 4.24 | 9.68 | 10.58 | 12.05 |
| p-Xylene | 0.18 | 0.21 | - | - |
| $C_9+$ | 1.36 | 0.29 | 0.57 | 0.45 |

## Claims

1. A synthetic porous crystalline material having an X-ray diffraction pattern including values set forth below:

14

EP 0 553 072 B1

| Interplanar d-Spacing (Å) | Relative Intensity, I/Io |
|---|---|
| 15.4 ± 0.23 | mw-m |
| 9.03 ± 0.14 | mw-ms |
| 6.62 ± 0.10 | w-mw |
| 4.99 ± 0.07 | mw-m |
| 4.04 ± 0.05 | m-ms |
| 3.31 ± 0.04 | mw-m |
| 2.00 ± 0.03 | mw-m |

2. The crystalline material of Claim 1 and having a composition comprising the molar relationship

$$X_2O_3:(n)YO_2$$

wherein n is at least 30, X is a trivalent element and Y is a tetravalent element.

3. The crystalline material of Claim 2 wherein n is from 40 to 200.

4. The crystalline material of Claim 2 or Claim 3 wherein X is aluminum and Y is silicon.

5. A method for preparing the synthetic porous crystalline material of Claim 1, said method comprising preparing a reaction mixture containing sources of alkali metal cations (M), a trivalent element (X) oxide, a tetravalent element (Y) oxide, hexamethyleneimine (R) and water, said trivalent element being selected from aluminum, boron, iron and gallium, said tetravalent element being selected from silicon and germanium, and said reaction mixture having a composition in terms of mole ratios within the following ranges:

| | |
|---|---|
| $YO_2/X_2O_3$ | = 50 to 200 |
| $H_2O/YO_2$ | = 10 to 100 |
| $OH^-/YO_2$ | = 0.01 to 0.2 |
| $M/YO_2$ | = 0.01 to 1.0 |
| $R/YO_2$ | = 0.1 to 1.0 |

and maintaining said reaction mixture under sufficient crystallization conditions until crystals of said material are formed.

6. The method of Claim 5 wherein said reaction mixture has a composition in terms of mole ratios within the following ranges:

| | |
|---|---|
| $YO_2/X_2O_3$ | = 50 to 150 |
| $H_2O/YO_2$ | = 15 to 40 |
| $OH^-/YO_2$ | = 0.02 to less than 0.1 |
| $K/YO_2$ | = 0.05 to 0.3 |
| $R/YO_2$ | = 0.1 to 0.5 |

7. The method of Claim 5 or Claim 6 wherein said crystallization conditions include a temperature of 80° to 250°C for a time of 24 hours to 20 days.

8. A process for converting an organic feedstock which comprises contacting said feedstock at organic compound conversion conditions with a catalyst comprising the synthetic porous crystalline material of Claim 1.

9. The process of Claim 8 wherein the conversion is alkylation of an aromatic compound.

15

EP 0 553 072 B1

**10.** The process of Claim 9 wherein the aromatic compound is benzene and is alkylated with ethylene.

**Patentansprüche**

**1.** Synthetisches poröses kristallines Material mit einem Röntgenbeugungsdiagramm, das die nachfolgenden Werte umfaßt:

| d-Netzebenenabstand (A) | Relative Intensität $I/I_o$ |
|---|---|
| $15,4 \pm 0,23$ | mw-m |
| $9,03 \pm 0,14$ | mw-ms |
| $6,62 \pm 0,10$ | w-mw |
| $4,99 \pm 0,07$ | mw-m |
| $4,04 \pm 0,05$ | m-ms |
| $3,31 \pm 0,04$ | mw-m |
| $2,00 \pm 0,03$ | mw-m |

**2.** Kristallines Material nach Anspruch 1, mit einer Zusammensetzung, die das Molverhältnis

$$X_2O_3:(n)Y_2$$

umfaßt, wobei n mindestens 30 ist, X ein dreiwertiges Element und Y ein vierwertiges Element ist.

**3.** Kristallines Material nach Anspruch 2, wobei n 40 bis 200 berägt.

**4.** Kristallines Material nach Anspruch 2 oder 3, wobei X Aluminium und Y Silicium ist.

**5.** Verfahren zur Herstellung des synthetischen porösen, kristallinen Materials nach Anspruch 1, wobei das Verfahren umfaßt: Herstellen einer Reaktionsmischung, die Quellen von Alkalimetallkationen (M), ein Oxid eines dreiwertigen Elements (X), ein Oxid eines vierwertigen Elements (Y), Hexamethylenimin (R) und Wasser enthält, wobei das dreiwertige Element aus Aluminium, Bor, Eisen und Gallium ausgewählt ist, das vierwertige Element aus Silicium und Germanium ausgewählt ist, und die Reaktionsmischung eine auf die Molverhältnisse bezogene Zusammensetzung innerhalb folgende Bereiche aufweist:

| | |
|---|---|
| $YO_2/X_2O_3$ | = 50 bis 200 |
| $H_2O/YO_2$ | = 10 bis 100 |
| $OH^-/YO_2$ | = 0,01 bis 0,2 |
| $M/YO_2$ | = 0,01 bis 1,0 |
| $R/YO_2$ | = 0,1 bis 1,0 |

und Halten der Reaktionsmischung bei ausreichenden Kristallisationsbedingungen, bis Kristalle des Materials gebildet wurden.

**6.** Verfahren nach Anspruch 5, wobei die Reaktionsischung eine auf die Molverhältnisse bezogene Zuammensetzung innerhalb der folgenden Bereiche aufweist:

| | |
|---|---|
| $YO_2/X_2O_3$ | = 50 bis 150 |
| $H_2O/YO_2$ | = 15 bis 40 |
| $OH^-/YO_2$ | = 0,02 bis weniger als 0,1 |
| $K/YO_2$ | = 0,05 bis 0,3 |
| $R/YO_2$ | = 0,1 bis 0,5 |

**7.** Verfahren nach Anspruch 5 oder Anspruch 6, wobei die Kristallisationsbedingungen eine Temperatur von 80 bis 250 °C während eines Zeitraums von 24 Stunden bis 20 Tagen umfassen.

16

EP 0 553 072 B1

8. Verfahren zur Umwandlung eines organischen Ausgangsmaterials, das den Kontakt des Ausgangsmaterials bei Umwandlungsbedingungen für die organische Verbindung mit einem Katalysator umfaßt, der das synthetische, poröse, kristalline Material nach Anspruch 1 umfaßt.

9. Verfahren nach Anspruch 8, wobei die Umwandlung die Alkylierung einer aromatischen Verbindung ist.

10. Verfahren nach Anspruch 9, wobei die aromatische Verbindung Benzol ist und mit Ethylen alkyliert wird.

**Revendications**

1. Un matériau cristallin poreux synthétique dont le spectre de diffraction aux rayons X présente les caractéristiques suivantes:

TABLEAU 1

| Distance interplanaire d (A) | Intensité relative, I/Io |
|---|---|
| $15,4 \pm 0,23$ | mw-m |
| $9,03 \pm 0,14$ | mw-ms |
| $6,62 \pm 0,10$ | w-mw |
| $4,99 \pm 0,07$ | mw-m |
| $4,04 \pm 0,05$ | m-ms |
| $3,31 \pm 0,04$ | mw-m |
| $2,00 \pm 0,03$ | mw-m |

2. Le matériau cristallin selon la revendication 1, et présentant une composition présentant la relation molaire

$$X_2O_3:(n)YO_2$$

dans laquelle n est égal à au moins 30, X est un élément trivalent et Y est un élément tétravalent.

3. Le matériau cristallin de la revendication 2, dans lequel n est compris entre 40 et 200.

4. Le matériau cristallin selon la revendication 2 ou la revendication 3, dans lequel X est de l'aluminium et Y est le silicium.

5. Un procédé de préparation d'un matériau cristallin poreux synthétique selon la revendication 1, ledit procédé comprenant la préparation d'un mélange réactionnel contenant des sources de cations de métal alcalin (M), un oxyde d'élément trivalent (X), un oxyde d'élément tétravalent (Y), de l'hexaméthylèneimine (R) et de l'eau, ledit élément trivalent étant choisi parmi aluminium, bore, fer, et gallium, ledit élément tétravalent étant choisi parmi silicium et germanium et ledit mélange réactionnel présentant la composition, en termes de rapport molaire, comprise dans les domaines suivants:

| | |
|---|---|
| $YO_2/X_2O_3$ | = 50 à 200 |
| $H_2O/YO_2$ | = 10 à 100 |
| $OH^-/YO_2$ | = 0,01 à 0,2 |
| $M/YO_2$ | = 0,01 à 1,0 |
| $R/YO_2$ | = 0,1 à 1,0 |

et le maintien dudit mélange réactionnel dans des conditions de cristallisation suffisantes jusqu'à ce que des cristaux dudit matériau soient formés.

6. Le procédé selon la revendication 5, dans lequel ledit mélange réactionnel présente la composition exprimée en rapport molaire, compris dans le domaines suivants:

17

| YO$_2$/X$_2$O$_3$ | = 50 à 150 |
|---|---|
| H$_2$O/YO$_2$ | = 15 à 40 |
| OH$^-$/YO$_2$ | = 0,02 à -0,1 |
| M/YO$_2$ | = 0,05 à 0,3 |
| R/YO$_2$ | = 0,1 à 0,5 |

7. Le procédé selon la revendication 5 ou la revendication 6, dans lequel lesdites conditions de cristallisation comprennent une température de 80 à 250°C et une durée de réaction de 24 heures à 20 jours.

8. Un procédé de conversion d'une charge organique qui comprend la mise en contact de ladite charge dans des conditions de conversion du composé organique, avec un catalyseur comprenant le matériau cristallin poreux synthétique selon la revendication 1.

9. Le procédé selon la revendication 8, dans lequel la conversion est une alkylation d'un dérivé aromatique.

10. Le procédé selon la revendication 9, dans lequel le dérivé aromatique est le benzène et l'alkylation est effectuée par l'éthylène.